# EUROPÄISCHE PATENTSCHRIFT

(11) **EP 1 511 534 B1**
(45) Veröffentlichungstag und Bekanntmachung des Hinweises auf die Patenterteilung: **06.07.2011**
(21) Anmeldenummer: 03735453.7
(22) Anmeldetag: 23.05.2003
(51) Int. Cl.: A61N 1/00

(54) **VORRICHTUNG ZUM ELEKTROCHIRURGISCHEN VERÖDEN VON KÖRPERGEWEBE**
DEVICE FOR ELECTROSURGICALLY DESTROYING BODY TISSUE
DISPOSITIF DESTINE A LA SCLEROSE ELECTROCHIRURGICALE DE TISSUS DE L'ORGANISME

(30) Priorität: 27.05.2002 DE 10224154; 10.04.2003 DE 10317243
(43) Veröffentlichungstag der Anmeldung: 09.03.2005
(73) Patentinhaber: Celon AG Medical Instruments, 14513 Teltow (DE)
(72) Erfinder: DESINGER, Kai, 12157 Berlin (DE); STEIN, Thomas, 14513 Teltow (DE); ROGGAN, André, 12163 Berlin (DE); PREZEWOVSKY, Thomas, 14513 Teltow (DE)
(74) Vertreter: Eisenführ, Speiser & Partner
(86) Internationale Anmeldenummer: PCT/EP2003/005439
(87) Internationale Veröffentlichungsnummer: WO 2003/099372

(56) Entgegenhaltungen:
- WO-A-02/32335
- US-A- 5 383 917
- US-A- 5 928 159
- US-A- 6 123 702
- US-A1- 6 092 528

## Beschreibung

Die vorliegende Erfindung betrifft eine Applikationsvorrichtung zum Applizieren eines Hochfrequenzstroms zum thermischen Veröden von Körpergewebe. Die Applikationsvorrichtung umfasst eine Elektrodenmenge mit mindestens drei in Körpergewebe einführbaren aktiven Elektroden. Außerdem umfasst die Applikationsvorrichtung einen Hochfrequenzgenerator zum Erzeugen einer Hochfrequenzspannung, der umschaltbar mit einer oder mehreren der Elektroden zu verbinden ist, und eine Messeinrichtung zum Messen der Impedanz des Körpergewebes zwischen allen oder ausgewählten aktiven Elektroden.

Das elektrochirurgische, insbesondere das elektrothermische Veröden von pathologisch verändertem Gewebe, im folgenden kurz Gewebe genannt, ist eine in der Medizin bekannte Methode. Von besonderem Interesse ist diese Methode für die Therapie von Organtumoren, zum Beispiel Lebertumoren. Zur Verödung werden eine oder mehrere Elektroden im zu verödenden Gewebe, d. h. dem Tumorgewebe, oder in dessen unmittelbarer Nähe platziert und ein Wechselstrom zwischen den Elektroden oder einer Elektrode und einer außen schen den Elektroden oder einer Elektrode und einer außen am Körper fixierten Neutralelektrode fließen lassen. Fließt der Strom zwischen der Elektrode und der Neutralelektrode (ggf. auch zwischen mehreren Elektroden und einer oder mehreren Neutralelektroden), so spricht man von einer monopolaren Elektrodenanordnung. Fließt der Strom dagegen zwischen den im Gewebe befindlichen Elektroden selbst (in diesem Fall müssen im Gewebe mindestens zwei Elektroden vorhanden sein), so spricht man von einer bipolaren Anordnung. Von einer multipolaren Anordnung spricht man, wenn im Gewebe mehr als zwei Elektroden vorhanden sind, zwischen denen Wechselstrom fließt.

Die zum Platzieren im Gewebe vorgesehenen Elektroden sind in der Regel als Elektrodennadeln ausgebildet. Sie besitzen einen elektrisch leitenden zylinderförmigen Schaft, der mit Ausnahme eines oder mehrerer distalen Bereiche, den sog. aktiven Bereichen der Elektrode oder kurz aktiven Elektroden, gegen das umliegende Gewebe elektrisch isoliert ist. Die aktiven Elektroden stehen dagegen mit dem Körpergewebe in elektrisch leitfähiger Verbindung. Optional sind die aktiven Elektroden auch mit integrierten Thermosensoren ausgestattet. In speziellen Ausführungsformen können am distalen Ende des Schaftes weitere aktive Elektroden, mechanisch ausgefahren werden, um das therapierbare Gewebevolumen zu vergrößern.

Mittels Hochfrequenzgenerator wird in der monopolaren Anordnung ein Stromfluss zwischen den aktiven Elektroden und der bzw. den Neutralelektroden induziert. In der alternativen bipolaren Anordnung kann auf die Neutralelektroden verzichtet werden. Der Stromkreis wird in diesem Fall über eine weitere aktive Elektrode geschlossen, wobei die erforderlichen aktiven Elektroden in einer koaxialen Anordnung gegeneinander isoliert auf der Elektrodennadel oder auf zwei getrennten Elektrodennadeln angeordnet sein können.

Durch den ohmschen Gewebewiderstand, der ein Teil der komplexen Gewebeimpedanz ist, erfolgt eine Umsetzung des über die Elektroden applizierten Wechselstroms in joulsche Wärme. Bei Temperaturen zwischen 50 und 100 °C kommt es zu einer massiven Denaturierung der körpereigenen Proteine (Koagulation) und in der Folge zum Absterben der betroffenen Gewebeareale. Aufgrund der hohen Stromdichte an den aktiven Elektroden erfolgt die Erwärmung vorwiegend im Bereich dieser Elektroden, so dass eine lokale thermische Tumordestruktion möglich ist.

Eine Vorrichtung und ein Verfahren zum elektrothermischen Veröden pathologischen Gewebes ist beispielsweise in US 5,630,426 oder WO 02/32335 offenbart.

Entscheidend für eine effektive und vor allem sichere Therapie ist die Ausbildung einer thermischen Destruktionszone, die der Ausdehnung des pathologischen Gewebes, d. h. des Tumorgewebes, optimal angepasst ist.

Aufgabe der vorliegenden Erfindung ist es, eine Vorrichtung zum elektrothermischen Veröden von Geweben zur Verfügung zu stellen, die eine zuverlässige Destruktion pathologischen Gewebes ermöglicht.

Diese Aufgabe wird durch eine Vorrichtung zum Applizieren eines Hochfrequenzstroms der eingangs genannten Art gelöst, die eine Auswahleinrichtung aufweist, die mit der Messeinrichtung verbunden ist und dazu ausgestaltet ist, eine mindestens zwei aktive Elektroden umfassende Untermenge aus der Elektrodenmenge anhand der gemessenen Impedanz auszuwählen. Die Vorrichtung zum Applizieren eines Hochfrequenzstroms weist außerdem eine Steuereinrichtung auf, die mit der Auswahleinrichtung verbunden ist und zum Anlegen der Hochfrequenzspannung an die aktiven Elektroden der ausgewählten Untermenge derart, dass zwischen ihnen ein Hochfrequenzstrom durch das Körpergewebe fließt, ausgestaltet ist.

In einer bevorzugten Anordnung ist die Applikationsvorrichtung dergestalt ausgeführt, dass der Hochfrequenzgenerator, die Messeinrichtung, die Auswahleinrichtung und die Steuereinrichtung in einem oder mehreren Gehäusen zu einem Applikationsgenerator zusammengefasst sind, der Steckanschlüsse für die Elektroden und deren Zuleitungen aufweist. Die Elektroden können somit wahlweise an den Applikationsgenerator angeschlossen werden.

Der Applikationsgenerator umfasst vorzugsweise eine Anschlusserkennungseinheit, die mit den Steckanschlüssen verbunden und ausgebildet ist, selbsttätig zu erkennen, dass an einem Steckanschluss eine Elektrode angeschlossen ist. Die Anschlusserkennungseinheit ist vorzugsweise mit der Steuereinheit verbunden.

Die Steuereinheit ist wiederum vorzugsweise so ausgebildet, dass alle angeschlossenen Elektroden in eine Elektrotherapie mit einbezogen werden, so dass mittels der Auswahlvorrichtung im Laufe einer vorgegebenen Zeit an jede angeschlossene Elektrode wenigstens einmal eine Hochfrequenzspannung angelegt wird.

In einer Ausführungsvariante können an dem Applikationsgenerator Schalter vorgesehen sein, mit denen bestimmte Elektroden wahlweise in dem Sinne abgeschaltet werden können, dass an eine abgeschaltete Elektrode keine Hochfrequenzspannung im vorgenannten Sinn angelegt wird.

Die weiteren Unteransprüche enthalten weitere vorteilhafte Ausgestaltungen der Erfindung.

Der Erfindung liegt die Erkenntnis zugrunde, dass für die Ausbildung einer optimal an das Tumorgewebe angepassten Destruktionszone die Elektrodenanordnung im Tumor eine entscheidende Rolle spielt. In der klinischen Praxis ist es mittlerweile üblich, bei größeren zu zerstörenden Gewebearealen mehrere Elektrodennadeln im Gewebe zu positionieren, um mittels Superposition der von den einzelnen Elektroden erzeugten thermischen Destruktionszonen eine Effizienzsteigerung zu erzielen. Darüber hinaus soll bei einigen Anwendungen durch eine mehrkanalige Temperaturmessung eine gleichmäßige thermische Destruktionszone erzielt werden. Dies hat sich in der Praxis jedoch als unzulänglich erwiesen, weil es sich nur um Punktmessungen handelt und die Gewebeeigenschaften in einiger Entfernung der Messstellen nicht berücksichtigt werden können. Damit kommt es immer wieder zu Untertherapie, d. h. in zu behandelnden Gewebearealen wird die für eine vollständige Destruktion des Tumorgewebes erforderliche Temperatur nicht erreicht. Infolgedessen erleiden die Patienten einen Rückfall und müssen sich erneut einer Therapie unterziehen.

Die Gewebeimpedanz hängt stark vom Fortschreiten der thermischen Gewebezerstörung ab. Mit zunehmender Verödung des Gewebes steigt insbesondere dessen ohmscher Widerstand und damit auch die Impedanz an. Der grundlegende Gedanke der Erfindung ist die Tatsache, dass im Gegensatz zur Temperatur die Gewebeimpedanz eine Volumeninformation darstellt, welche die Gewebeeigenschaften zwischen den beiden für die Impedanzmessung nötigen Messstellen integral beschreibt. Erfindungsgemäß soll daher die Änderung des Impedanzverhaltens in Kombination mit einer multipolaren Applikationsanordnung zur optimalen Steuerung der Therapie genutzt werden. Überraschenderweise zeigt sich, dass bei einer Mehrfachapplikation, d.h. dem Fließenlassen von Hochfrequenzströmen zwischen mehreren aktiven Elektrodenpaaren, die Gewebeimpedanz zwischen den einzelnen aktiven Elektroden keinen einheitlichen Verlauf aufweist. Vielmehr kann das Gewebe zwischen einzelnen aktiven Elektrodenpaaren bereits in den Austrocknungszustand übergegangen sein, wobei es eine sehr hohe Impedanz aufweist, während das Gewebe zwischen anderen aktiven Elektrodenpaaren diesen Zustand noch nicht erreicht hat und demgemäss eine sehr niedrige Impedanz aufweist. Dies korreliert mit der klinischen Erkenntnis, dass auch bei punktueller Temperaturmessung eine sichere Volumendestruktion nicht garantiert werden kann. Ursache dieser nichthomogenen Austrocknung des Körpergewebes sind beispielsweise nicht gleichmäßig verteilte Blutgefässe, die eine lokal begrenzte Kühlwirkung ausüben und damit dem Therapieeffekt entgegenwirken Außerdem dem kann bei einer Mehrelektrodenkonfiguration, ähnlich wie bei einer rein bipolaren Anordnung, auf die Neutralelektrode oder die Neutralelektroden ganz verzichtet werden. Der Stromfluss bleibt dadurch auf die Zielregion beschränkt, Nebenwirkungen, wie sie von der monopolaren Anwendung bekannt sind, können daher nicht mehr auftreten.

Die Messeinheit ist vorzugsweise ausgebildet, aus einer gemessenen Impedanz den ohmschen Widerstand zwischen einer vorgegebenen Anzahl aktiver Elektroden (5, 6) einer Untermenge (19) aller angeschlossenen Elektroden abzuleiten. Dies geschieht vorzugsweise durch eine Bestimmung des ohmschen oder Wirkwiderstandes R für kleine Werte von R (0-200 Ohm), in dem der Quotient aus einer gemessenen Wirkleistung und dem Quadrat eines gemessenen Stromes gebildet wird. Für große Werte von R (ab ca. 4200 Ohm) wird R hingegen maßgeblich durch den Quotienten zwischen dem Quadrat einer gemessenen Spannung und einer gleichzeitig gemessenen Leistung bestimmt. Der Vorteil dieser Ausführungsvariante besteht darin, dass die Steuerung der Leistung für eine Gewebeablation maßgeblich von dem tatsächlich interessierenden ohmschen Gewebewiderstand abhängig erfolgen kann, während eine Steuerung in Abhängigkeit der gemessenen Impedanz stärker von einem variablen Blindwiderstandanteil z.B. aufgrund von Leitungskapazitäten und -induktivitäten beeinträchtigt ist. Ziel der Bestimmung des wirksamen ohmschen Widerstandes ist es, den Einfluss des Blindwiderstands auf die Ansteuerung der Elektroden mit einfachen Mitteln zu reduzieren.

Die erfindungsgemäße Applikationsvorrichtung zum Applizieren eines Hochfrequenzstroms zum thermischen Veröden von Körpergewebe umfasst eine Elektrodenmenge mit mindestens drei in Körpergewebe einführbare aktive Elektroden, einen Hochfrequenzgenerator zum Erzeugen einer Hochfrequenzspannung, der umschaltbar mit einer oder mehreren der aktiven Elektroden zu verbinden ist, und eine Messeinrichtung zum Messen der Impedanz oder des ohmschen Widerstandes des Gewebes zwischen allen oder ausgewählten aktiven Elektroden. Außerdem umfasst die Applikationsvorrichtung eine Auswahleinrichtung, die mit der Messeinrichtung verbunden ist und ausgestaltet ist, eine mindestens zwei aktive Elektroden umfassende Untermenge aus der Elektrodenmenge anhand der gemessenen Impedanz bzw. des ohmschen Widerstandes auszuwählen. Des weiteren ist eine Steuereinrichtung vorhanden, die mit der Auswahleinrichtung verbunden und zum Anlegen der Hochfrequenzspannung an die aktiven Elektroden der ausgewählten Untermenge derart, dass zwischen ihnen ein Hochfrequenzstrom durch das Körpergewebe fließt, ausgestaltet ist.

Als Untermenge ist hierbei jede mindestens zwei aktive Elektroden umfassende Teilmenge der Elektrodenmenge zu verstehen, einschließlich des Falles, dass als Untermenge die gesamte Elektrodenmenge ausgewählt wird. Da die Impedanz und insbesondere der ohmsche Widerstand als Volumeninformation über die Gewebeeigenschaften ein Maß für den Fortschritt der Verödung darstellt, ist die Impedanz- bzw. Wirkwiderstandsmessung geeignet, unterschiedliche Verödungsgrade in Bereichen des zu verödenden Gewebes festzustellen. Die verschiedenen Gewebebereiche können dann durch das Anlegen der Hochfrequenzspannung an ausgewählte aktive Elektroden der Elektrodenmenge gezielt behandelt werden. Die Auswahl der Elektroden, d.h. das Bilden der Untermenge, bestimmt dabei die Strompfade durch das zu verödenden Gewebe.

In einer Ausgestaltung der Erfindung ist die Auswahleinrichtung derart ausgestaltet, dass eine neue Impedanz- bzw. Wirkwiderstandmessung und eine neue Auswahl einer mindestens zwei aktive Elektroden umfassenden Untermenge vorgenommen wird, wenn seit der vorangegangenen Auswahl eine vorbestimmte Zeit verstrichen ist.

Durch das Applizieren des Hochfrequenzstromes nur über eine vorbestimmte Zeit und das Neuauswählen der Untermenge anhand einer neuen Impedanz- bzw.- Wirkwiderstandsmessung lässt sich der Fortschritt des Verödungsprozesses in vorbestimmten Abständen überprüfen und die weitere Applikation an den festgestellten Fortschritt anpassen. Die Neuauswahl bietet dabei die Möglichkeit, eine andere Untermenge von Elektroden als die bisher verwendete Untermenge auszuwählen und so auf die fortschreitende Verödung mit veränderten Strompfaden im zu verödenden Gewebe zu reagieren. Dies ist insbesondere von Vorteil, wenn die Verödung nicht im gesamten zu verödenden Gewebe gleichmäßig erfolgt, sondern lokal mit unterschiedlicher Geschwindigkeit voranschreitet. Außerdem lassen sich durch die zeitliche Begrenzung der Applikation Verbrennungen der behandelten Gewebebereiche durch zu lange Applikation des Hochfrequenzstromes vermeiden.

In einer weiteren Ausgestaltung der Erfindung ist die Messeinrichtung dazu ausgelegt, die Impedanz bzw. den Wirkwiderstand des Körpergewebes während der Applikation des Hochfrequenzstroms zu messen. Die gemessenen Impedanzen bzw. Wirkwiderstandswerte können z.B. mit Referenzwerten, die vor Beginn der elektrothermischen Behandlung gemessen worden sind, in Beziehung gesetzt werden. Diese Ausgestaltung bietet den Vorteil, dass anhand der Impedanz- bzw. Wirkwiderstandsmessung am Gewebe zwischen den Elektroden, insbesondere anhand der Änderung der Impedanz- bzw. Wirkwiderstandswerte gegenüber den Referenzwerten, der Fortschritt der Verödung laufend erfasst wird. Die Dauer der Applikation des Hochfrequenzstroms über die Elektrodenuntermenge kann so abhängig vom Fortschreiten der Verödung bestimmt werden. Durch den Vergleich der gemessenen Impedanz- bzw. Wirkwiderstandswerte mit den vorab bestimmten Referenzwerten lassen sich unerwünschte Einflüsse auf die gemessenen Impedanz- bzw. Wirkwiderstandswerte, die bspw. aus unterschiedlichen Abständen zwischen den verschiedenen Elektroden oder aus bereits vor Beginn der Behandlung vorherrschenden unterschiedlichen Impedanzen verschiedener zu behandelnder Gewebebereiche resultieren, beim Bestimmen der Applikationsdauer berücksichtigen.

Vorteilhafterweise ist die Auswahleinrichtung derart ausgestaltet, dass eine neue Auswahl einer mindestens zwei aktive Elektroden umfassenden Untermenge vorgenommen wird, wenn die Impedanz oder der Wirkwiderstand des Körpergewebes zwischen einer vorgegebenen Anzahl aktiver Elektroden der Untermenge einen vorbestimmten Wert erreicht oder überschreitet.

Das Erreichen oder Überschreiten eines vorbestimmten Impedanz- bzw. des Wirkwiderstandswertes zeigt an, dass die Verödung bis zu einem bestimmten Grad fortgeschritten ist. Die Verödung kann dann an einem anderen, noch nicht so weit verödeten Teil des zu verödenden Gewebes fortgesetzt werden. Damit lassen sich unnötig lange Applikationsdauern und daraus resultierende Belastungen des Patienten vermeiden. Daneben lassen sich auch Verbrennungen des Gebwebes durch zu lange Applikation des Hochfrequenzstromes an einem Gewebebereich besonders effektiv vermeiden.

Die zeitabhängige und die impedanzabhängige Auswahl können auch kombiniert werden, so dass eine Neuauswahl immer dann stattfindet, wenn eine vorbestimmte Zeit verstrichen oder eine vorbestimmte Impedanz oder ein vorbestimmter Wirkwiderstand erreicht ist.

In noch einer weiteren Ausgestaltung der Erfindung ist die Auswahleinrichtung derart ausgestaltet, dass sie vor der Auswahl bzw. der Neuauswahl der mindestens zwei aktive Elektroden umfassenden Untermenge die Messeinrichtung veranlasst, eine Messung der Impedanz oder des Wirkwiderstandes des Körpergewebes zwischen allen möglichen Paaren aus aktiven Elektroden durchzuführen, und als Untermenge diejenigen aktiven Elektroden auswählt, zwischen denen die Impedanz oder der Wirkwiderstand am geringsten ist oder einen vorbestimmten Wert nicht überschreitet.

Dadurch, dass bei der Neuauswahl diejenigen aktiven Elektroden ausgewählt werden, zwischen denen das Körpergewebe die geringste Impedanz oder den geringsten Wirkwiderstand aufweist, lässt sich die Hochfrequenzspannung gezielt auf diejenigen Gewebebereiche anwenden, in denen die Verödung am wenigsten weit fortgeschritten ist.

Eine vorteilhafte Weiterbildung der erfindungsgemäßen Applikationsvorrichtung zeichnet sich dadurch aus, dass die Steuervorrichtung derart ausgestaltet ist, dass zu Beginn der Applikation die Hochfrequenzspannung in einem vorgegebenen zyklischen Wechsel an die aktiven Elektroden angelegt wird und die Auswahl der mindestens zwei aktive Elektroden umfassenden Untermenge anhand der Impedanz oder des Wirkwiderstandes zu einem spätem Zeitpunkt der Applikation einsetzt. Zu beginn der Applikation besitzt die Impedanz bzw. der Wirkwiderstand im gesamten zu verödenden Gewebe häufig noch einen gleichen oder annähernd gleichen Wert. Die lokalen Unterschiede in der Impedanz oder im Wirkwiderstand stellen sich erst im Verlauf der Applikation ein, so dass eine impedanz- bzw. wirkwiderstandsangepasste Auswahl der Elektroden erst im Verlauf der Applikation sinnvoll wird.

In einer weiteren vorteilhaften Weiterbildung zeichnet sich die Applikationsvorrichtung dadurch aus, dass die Auswahleinrichtung derart ausgestaltet ist, dass als Untermenge mindestens drei aktive Elektroden ausgewählt werden und die Steuereinrichtung derart ausgestaltet ist, dass die aktiven Elektroden mit Hochfrequenzspannungen beaufschlagt werden, die jeweils um einen festen Phasenwinkel zueinander phasenverschoben sind. Der phasenverschobene Hochfrequenzstrom führt zu einer verbesserten Homogenität des applizierten Hochfrequenzstroms im zu verödenden Gewebe.

Vorteilhafterweise werden als Untermenge drei aktive Elektroden ausgewählt, wobei die Phasenwinkel 120 Grad betragen. Dies ermöglicht es, die aktiven Elektroden mit Dreiphasenstrom, d.h. Drehstrom, zu betreiben.

Die Auswahleinrichtung oder die Steuereinrichtung ist vorzugsweise derart ausgestaltet, dass sie Untermengen oder Elektrodenkombinationen, für die die Impedanz oder der ohmsche Widerstand einen vorgegebenen Maximalwert Rₘₐₓ überschreitet, abschaltet. Die Gewebeverödung ist dann in dem entsprechenden Volumenelement beendet.

Die Steuereinrichtung ist zusätzlich oder alternativ ausgebildet, eine über eine jeweils ausgewählte Untermenge vorgegebene abzugebende Maximalleistung Pₘₐₓ bei Überschreiten einer definierten Impedanz oder eines definierten ohmschen Widerstandes kleiner Rₘₐₓ auf ein vorgegebenes Maß zu reduzieren. Damit wird kurz vor Abschluss der Verödung ein vorzeitiges Abschalten der ausgewählten Untermenge der Elektroden aufgrund reinen Austrocknens des weitgehend verödeten Gewebes verhindert.

Die Auswahleinrichtung oder die Steuereinrichtung ist vorzugsweise derart ausgestaltet, dass sie Untermengen oder Elektrodenkombinationen, für die die Impedanz oder der ohmsche Widerstand einen vorgegebenen Minimalwert Rₘᵢₙ unterschreitet, abschaltet. Aus diese Weise lassen sich Kurzschlüsse erkennen und eine Gefährdung des Patienten verhindern.

Die Steuereinrichtung stellt vorzugsweise eine über eine jeweils ausgewählte Untermenge abgegebene Leistung nach dem Auswählen der Untermenge zunächst geringer ein und erhöht die Leistung innerhalb vorgegebener Zeit in einer oder mehreren Stufen oder gleitend auf eine vorgegebene Maximalleistung Pₘₐₓ. Damit wird ein 'Popcorn Effekt' genannter Effekt vermieden, der darauf beruht dass bei schlagartig applizierter Maximalleistung mögliche Dampfblasen nicht schnell genug abgeleitet werden, so dass durch den plötzlich entstehenden Druck das Gewebe ruptiert oder von der Elektrodennadel abhebt.

Die aktiven oder ausgewählten Elektroden der Applikationsvorrichtung sind in einer vorteilhaften Ausgestaltung der Erfindung an Elektrodennadeln angeordnet, die das genaue Anordnen der aktiven Elektroden im zu verödenden Gewebebereich oder um den zu verödenden Gewebebereich herum ermöglichen.

Die Elektrodennadeln können als bipolare Elektrodennadeln ausgestaltet sein, also jeweils zwei voneinander isolierte aktive Elektroden umfassen, die unabhängig voneinander mit Hochfrequenzspannung beaufschlagt werden können. Dadurch ist es möglich, den Hochfrequenzstrom nicht nur zwischen verschiedenen Elektrodennadeln fliesen zu lassen, sondern auch entlang einer einzelnen Elektrodennadeln, was die Variationsmöglichkeit der Strompfade und damit die Anzahl der individuell verödbaren Teilbereiche des zu verödenden Gewebebereiches erhöht.

Die Variationsmöglichkeiten können durch die Verwendung multipolarer Elektrodennadeln, d.h. Elektrodennadeln mit mehr als zwei aktiven Elektroden, weiter vermehrt werden.

Eine vorteilhafte Weiterbildung der Elektrodennadein zeichnet sich dadurch aus, dass sie mit einer isolierenden Umhüllung umgeben sind, aus der sie um vorbestimmte Längen ausfahrbar sind, so dass die Anzahl der Elektroden der multipolaren Elektrodennadeln durch Aus- und Einfahren der Elektrodennadeln aus der Umhüllung einstellbar ist.

Eine Elektrodennadel kann einen oder mehrere Kanäle für ein Fluid zur Kühlung oder Heizung der aktiven Elektroden oder der gesamten Nadel aufweisen. Als Fluid kommen insbesondere Gase oder Flüssigkeiten in betracht. Die Heizung bzw. Kühlung kann dazu Verwendung finden, die durch den Hochfrequenzstrom erfolgende Erwärmung des Gewebes, d.h. den Temperaturverlauf im Gewebe, zu homogenisieren. Die Kanäle für das Fluid lassen sich auch in den bisher üblichen Elektrodennadeln zum Erzielen eines homogeneren Temperaturverlaufs einsetzen.

In einer Ausgestaltung zeichnen sich die Kanäle dadurch aus, dass sie zu den aktiven Elektroden führen und zum Zuführen von Kühlfluid geeignet sind. Die Erwärmung des Gewebes ist dort am stärksten, wo die Stromdichte des Hochfrequenzstroms am höchsten ist. Typischerweise ist die Stromdichte an den aktiven Elektroden höchsten. Die Kühlung der Elektroden kann die Temperatur des unmittelbar an die Elektroden angrenzenden Gewebes herabsetzen und so zu einem homogeneren Verlauf der Temperatur im Gewebe führen.

In einer Ausführungsform ist das Fluid eine entionisierte Flüssigkeit. Flüssigkeiten haben typischerweise einen höheren Wärmekoeffizienten als Gase, sind jedoch in der Regel elektrisch leitfähig, so dass für eine gute elektrische Isolation der Kanäle gesorgt werden muss. Durch das Verwenden von entionisierten und daher nicht leitenden Flüssigkeiten kann auf die elektrische Isolation der Kanäle weitgehend verzichtet werden. Je besser die Entionisierung der Flüssigkeit ist, desto weniger aufwändig braucht die Isolation zu sein.

Weitere Merkmale und Vorteile der vorliegenden Erfindung werden nachfolgend anhand eines detaillierten Ausführungsbeispiels unter Bezugnahme auf die beiliegenden Zeichnungen beschrieben.
- Figur 1: zeigt in einem Blockschaltbild die Komponenten der vor- liegenden Erfindung.
- Figur 2: zeigt das Blockschaltbild einer alternativen Ausgestal- tung der Erfindung.
- Figuren 3a und 3b: zeigen eine Elektrodennadel zur Verwendung in der erfindungsgemäßen Vorrichtung.
- Figuren 4a und 4b: zeigen eine beispielhafte Anordnung der Elektrodenna- deln im Gewebe in Draufsicht und Seitenansicht sowie die zugehörigen Stromflüsse.
- Figuren 5a und 5b: zeigen eine weitere beispielhafte Anordnung der Elekt- rodennadeln im Gewebe in Draufsicht und Seitenansicht sowie die zugehörigen Stromflüsse.

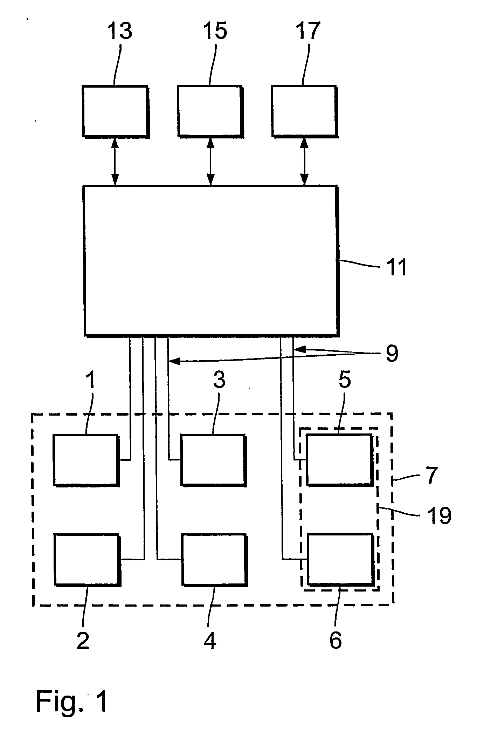

Figur 1 ist ein Blockschaltbild eines Ausführungsbeispieles für die erfindungsgemäße Vorrichtung zum Veröden von Körpergeweben. Die Vorrichtung umfasst eine Anzahl von aktiven Elektroden 1 bis 6, die zusammen eine Elektrodenmenge 7 bilden. Die Elektroden 1 bis 6 sind jeweils über individuelle Leitungen 9 mit einer Steuereinheit 11 verbunden. Mit der Steuereinheit 11 sind wiederum eine Messeinrichtung 13 zum Messen der Impedanz oder des Wirkwiderstandes des Gewebes zwischen Elektroden 1 bis 6, eine Auswahleinrichtung 15 zum Auswählen von Elektroden und ein Hochfrequenzgenerator 17 zum Erzeugen hochfrequenter Wechselspannungen verbunden.

Der Hochfrequenzgenerator 17 hat bei multipolarer Anwendung beispielsweise eine Ausgangsleistung von 250 W an 20 - 50 Ω, bei einer Arbeitsfrequenz von 470 kHz. Im Falle bipolarer Anwendungen beträgt die Ausgangsleistung beispielsweise 125 W an 100 Ω, ebenfalls bei einer Arbeitsfrequenz von 470 kHz. Der Arbeitsbereicht des Hochfrequenzgenerators liegt zwischen 10 und 1.000 Ω. Die Impedanz- bzw. Wirkwiderstandsmessung durch die Messeinrichtung 13 kann entweder bei der Arbeitsfrequenz von 470 kHz erfolgen, oder aber bei einer anderen Frequenz, insbesondere einer niedrigeren Frequenz, zum Beispiel 20 kHz. Der Hochfrequenzgenerator ist derart ausgelegt, dass er für typische Applikationszeiten von 20 Minuten und länger bei Volllast geeignet ist.

Die Messeinrichtung 13 ist derart ausgestaltet, dass sie in der Lage ist, die Impedanz bzw. den Wirkwiderstand des Gewebes zwischen jeweils zwei Elektroden der Elektrodenmenge 7 zu messen. Der Messvorgang kann beispielsweise erfolgen, indem zwischen zwei Elektroden der Elektrodenmenge 7 eine Wechselspannung angelegt wird und die Messeinrichtung 13 dann den durch die beiden Elektroden fließenden Wechselstrom misst. Dabei ist es ausreichend, den Betrag der Impedanz, also den Wirkwiderstand des Gewebes, zu messen. Alternativ kann die Messeinrichtung 13 jedoch auch den Phasenwinkel zwischen dem durch die beiden Elektroden fließenden Wechselstrom und der an den Elektroden anliegenden Wechselspannung ermitteln, so dass sich die Impedanz vollständig bestimmen lässt. Aus dem Ergebnis der Impedanzmessung kann die Auswahleinrichtung 15 den Verödungsgrad des Gewebes zwischen den beiden Elektroden ermitteln. Je höher der Verödungsgrad ist, desto höher ist die Impedanz des Gewebes.

Um die Frequenzabhängigkeit der Impedanz beim Bestimmen des Gewebezustandes berücksichtigen zu können, kann die Impedanzmessung bei mehreren Frequenzen vorgenommen werden. Die in der Frequenzabhängigkeit der Impedanz enthaltene Information kann beispielsweise beim Bestimmen von Applikationsparametern für die Applikation des Hochfrequenzstromes Berücksichtigung finden.

Die beschriebene Impedanzmessung erfolgt im vorliegenden Ausführungsbeispiel für alle möglichen Elektrodenpaare der Elektrodenmenge 7, so dass der Auswahleinrichtung 15 lmpedanzwerte für alle Gewebebereiche zwischen jeweils zwei Elektrodenpaaren zur Verfügung stehen. Anhand dieser Impedanzwerte wählt die Auswahleinrichtung 15 dann mindestens zwei Elektroden der Elektrodenmenge 7 aus, die eine Untermenge der Elektrodenmenge 7 bilden.

Die ausgewählte Untermenge teilt die Auswahleinrichtung 15 der Steuereinheit 11 mit, die dann über die Leitungen 9 den Elektroden der Untermenge 19, hier die Elektroden 5 und 6, die vom Hochfrequenzgenerator 17 erzeugte Hochfrequenzspannung zuführt. Dazu umfasst die Steuereinheit 11 eine Schalteinheit, mit deren Hilfe die Leitungen 9 individuell mit dem Hochfrequenzgenerator 17 verbindbar sind. Der Hochfrequenzgenerator 17 kann beispielsweise eine Konstantstrom- oder Konstantspannungsquelle sein.

Wenn die Hochfrequenzspannung an die Elektroden 5 und 6 der Untermenge 19 angelegt wird, fließt zwischen ihnen ein hochfrequenter Wechselstrom, der zu einer Erwärmung des Gewebes und als Folge dessen zu einer Denaturierung des Gewebes führt. Nach einer vorgegebenen Zeitdauer beendet die Steuereinheit 11 das Zuführen der Hochfrequenzspannung an die Elektroden der Untermengen 19 und veranlasst die Messeinrichtung 13, erneut die Impedanz zwischen allen möglichen Elektrodenpaaren der Elektrodenmenge 7 zu messen. Die Auswahleinrichtung 15 wählt dann beispielsweise aus der Elektrodenmenge das Elektrodenpaar als Untermenge 19 aus, zwischen dem das Gewebe den geringsten Impedanzanstieg aufweist. Die Impedanzwerte können ggf. auch mit Gewichtungsfaktoren unterschiedlich gewichtet werden. Das ausgewählte Elektrodenpaar kann dasselbe Elektrodenpaar 5, 6 wie im vorangegangenen Auswahlprozess oder ein anderes Elektrodenpaar sein. Letzteres wird der Fall sein, wenn aufgrund der elektrothermischen Behandlung die Impedanz des Gewebes zwischen den Elektroden 5 und 6 derart angestiegen ist, dass sie höher ist als die Impedanz des Gewebes zwischen mindestens einem anderen Elektrodenpaar.

Eine Untermenge 19 der Elektrodenmenge 7 kann auch mehr als zwei Elektroden umfassen. In diesem Fall kann es sinnvoll sein, eine Impedanzschwelle vorzugeben, die vorgibt, ob dem Gewebe zwischen den entsprechenden Elektrodenpaaren ein Hochfrequenzstrom zugeführt werden soll oder nicht. Erreicht oder überschreitet die Impedanz des Gewebes zwischen einem Elektrodenpaar die Impedanzschwelle nicht, so wird das betreffende Elektrodenpaar in die Untermenge 19 aufgenommen.

In einer alternativen Ausgestaltung der Erfindung misst die Messeinrichtung 13 während der Applikation des Hochfrequenzstroms kontinuierlich die Impedanz zwischen den Elektroden und gibt ein Signal an die Steuereinrichtung 11 aus, sobald die Impedanz oder der Impedanzanstieg des Gewebes zwischen den Elektroden 5 und 6 einen vorbestimmten Schwellenwert überschreitet. Die Steuereinrichtung beendet dann die Applikation des Hochfrequenzstroms. Nach dem Beenden der Applikation des Hochfrequenzstromes veranlasst die Steuereinrichtung 11 die Messeinrichtung 13, erneut die Impedanz des Gewebes zwischen allen Elektrodenpaaren zu messen, und veranlasst danach die Auswahleinrichtung 15 anhand des Ergebnisses der Impedanzmessung eine neue Untermenge 19 der Elektrodenmenge 7 auszuwählen, über die dann dem Gewebe der Hochfrequenzstrom zugeführt wird.

Umfasst die Untermenge 19 mehr als zwei Elektroden, so kann es vorgesehen sein, dass die Steuereinrichtung 11 die Applikation des Hochfrequenzstroms beendet, sobald die Impedanz des Gewebes zwischen einem der Elektrodenpaare einen vorbestimmten Wert überschreitet oder alternativ dann, wenn bei einer vorbestimmten Anzahl von Elektrodenpaaren das dazwischen liegende Gewebe die vorgegebene Impedanzschwelle überschreitet.

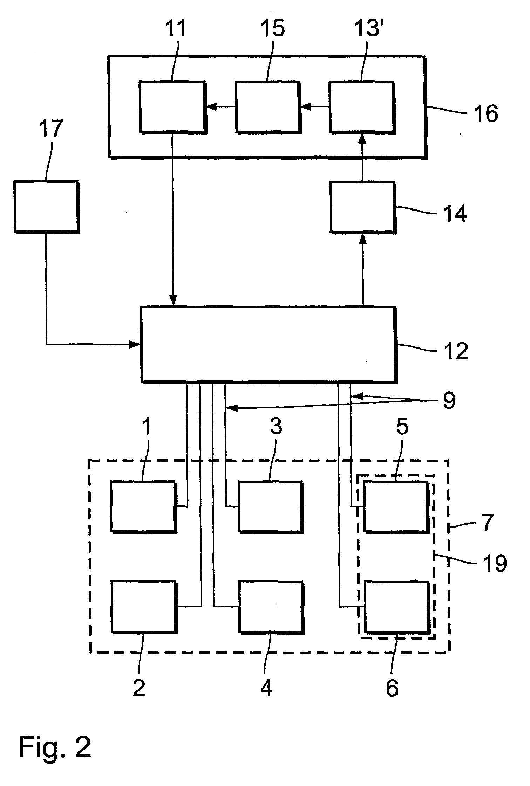

Eine alternative. Ausgestaltung der Erfindung ist als Blockschaltbild in Figur 2 dargestellt. Gleiche Elemente wie in der in Figur 1 dargestellten Ausführungsform sind mit den gleichen Bezugszeichen bezeichnet und werden im Folgenden nicht weiter erläutert.

Die alternative Ausgestaltung unterscheidet sich von der in Figur 1 dargestellten dadurch, dass eine Schalteinheit 12 vorhanden ist, die als von der Steuereinheit 11 getrennte Einheit ausgebildet ist. Mit der Schalteinheit 12 direkt verbunden ist der Hochfrequenzgenerator 17 sowie ein A/D-Wandler 14 zum Umwandeln der aus der Impedanzmessung resultierenden analogen Signale in digitale Signale, die an einen Prozessor 16 abgegeben werden. Die Schalteinheit 12 ist dazu ausgelegt, jede Elektrode 1-6 individuell mit dem Hochfrequenzgenerator 17 und/oder dem A/D-Wandler 14 zu verbinden.

Der Prozessor 16 umfasst die Mess- oder Auswerteeinheit 13', die mit dem A/D-Wandler 14 zum Empfang der aus der Impedanzmessung resultierenden digitalen Signale verbunden ist und aus den empfangenen Signalen die Impedanz des Körpergewebes ermittelt. Die Auswerteeinheit 13' ist außerdem zum Ausgeben der Impedanzwerte mit der Auswahleinheit 15 verbunden, die wiederum mit der Steuereinheit 11 verbunden ist. Die Auswahleinheit 15 wählt die zu verwendenden aktiven Elektroden (im dargestellten Ausführungsbeispiel die Elektroden 5 und 6) anhand der Impedanzwerte aus und teilt die Auswahl dann der Steuereinheit 11 mit. Gemäß dieser Auswahl wirkt die Steuereinheit 11 über eine Steuerleitung auf die Schalteinheit 12 ein, so dass sie die ausgewählten aktiven Elektroden mit dem Hochfrequenzgenerator 17 verbindet.

Alternativ kann die Steuereinheit 11 zusätzlich über eine Steuerleitung mit dem Hochfrequenzgenerator 17 verbunden sein, um die Frequenz der vom Hochfrequenzgenerator 17 abgegebenen Hochfrequenzspannung einstellen zu können.

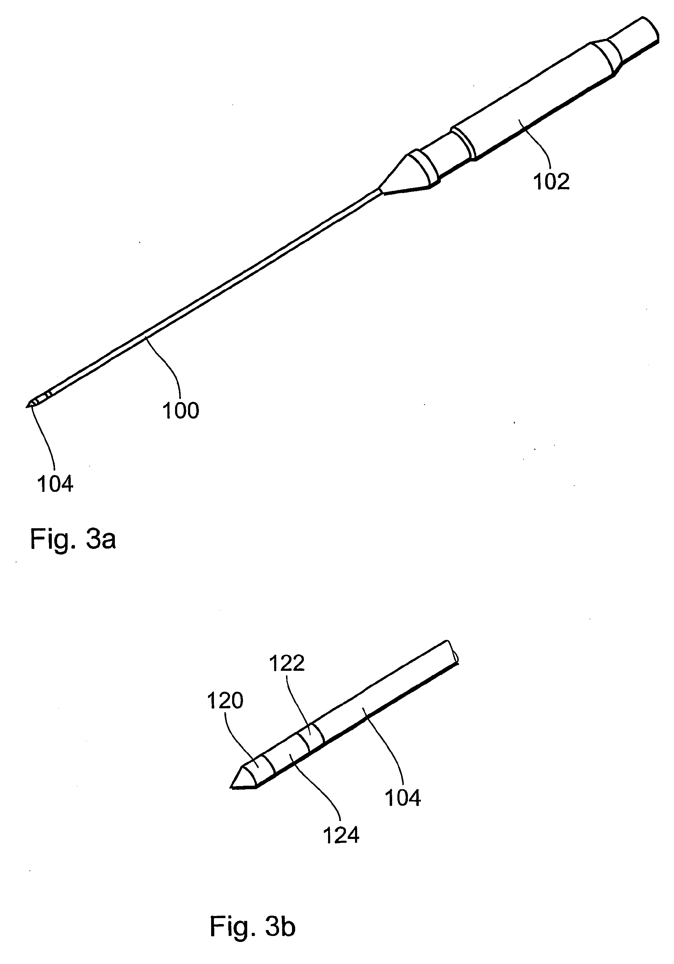

In den dargestellten Ausführungsformen sind die aktiven Elektroden als Elektrodennadeln ausgeführt. Eine solche Elektrodennadel ist in der Figur 3a gezeigt. Die Elektrodennadel 100 weist an ihrem proximalen Ende, d. h. dem aus dem Gewebe herausragenden Ende, einen Griffbereich 102 und an ihrem distalen Ende, d. h. dem zum Einbringen in das Gewebe vorgesehenen Ende 104 zwei aktive Elektroden 120 und 122 auf (siehe Figur 3b). Zwischen den beiden Elektroden 120 und 122 befindet sich ein isolierender Bereich 124, der die beiden Elektroden voneinander elektrisch isoliert. Obwohl die in Figur 3a und 3b dargestellte Elektrodennadel zwei Elektroden aufweist, kann die Elektrodennadel auch mehr als zwei aktive Elektroden oder nur eine Elektrode aufweisen. Sind mehr als zwei Elektroden vorhanden, so befindet sich zwischen allen Elektroden jeweils ein isolierender Bereich. Alle Elektroden einer Elektrodennadel 100 sind jeweils über individuelle Leitungen mit einem Steuergerät (nicht gezeigt) derart verbunden, dass jeder Elektrode individuell eine Hochfrequenzspannung zugeführt werden kann.

Die Elektrodennadel 100 kann in ihrem Inneren auch einen oder mehrere Kanäle zum Zuführen eines Fluids umfassen, um die aktiven Elektroden oder die gesamte Nadel zu kühlen oder zu heizen.

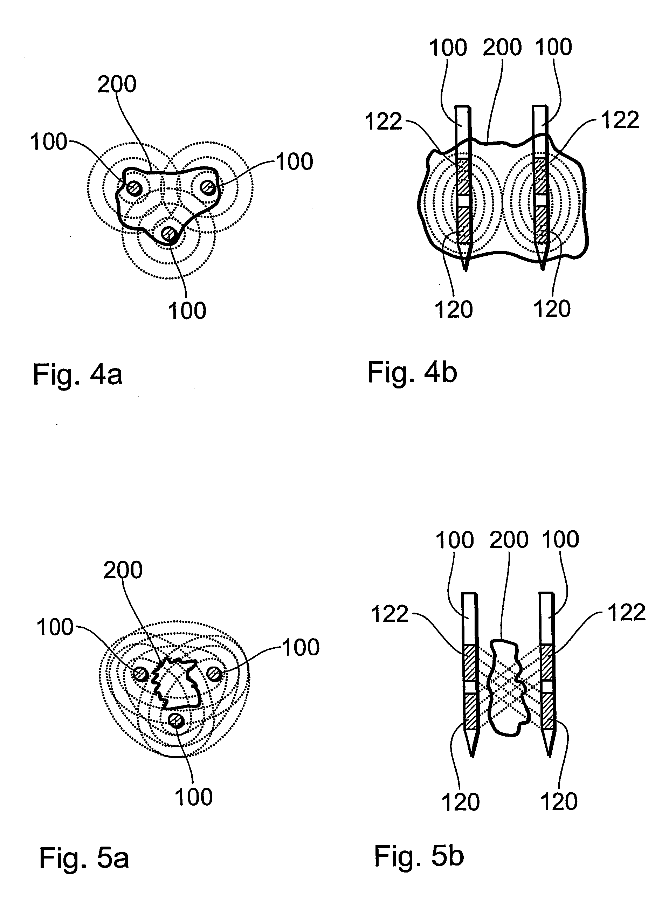

In den Figuren 4a und 4b ist ein erstes Beispiel für die Anwendung der Applikationsvorrichtung schematisch dargestellt. Die Figuren zeigen ein pathologisches Gewebe 200, das Zielgewebe, in das drei Elektrodennadeln 100 derart punktiert sind, dass ihre aktiven Elektroden 120 und 122 in direktem elektrischen Kontakt mit dem Zielgewebe stehen.

Die Elektroden 120 und 122 werden von der Steuereinheit 11 zuerst derart mit einer Hochfrequenzspannung beaufschlagt, dass ein Hochfrequenzstrom entlang den Elektrodennadeln 100 in axialer Richtung zwischen den Elektroden 120 und 122 fließt. Diese Stromflüsse sind in Figur 4b, die entsprechenden Potentiale in Figur 4a durch punktierte Linien angedeutet.

Figur 4a zeigt eine Draufsicht auf die drei Elektrodennadeln 100, während Figur 4b eine Seitenansicht von zwei der drei Elektrodennadeln 100 zeigt. Durch die Überlagerung der Stromflüsse aller drei Elektrodennadeln 100 entsteht zwischen den Nadeln ein Gewebebereich mit homogenem Stromfluss, so dass sich eine homogene Erwärmung des Gewebebereiches erzielen lässt.

Gleichzeitig mit der Applikation des Hochfrequenzstromes wird bei der Arbeitsfrequenz oder aber einer anderen Frequenz von der Messeinrichtung 13 die Gewebeimpedanz zwischen den Elektroden 120 und 122 gemessen. Insbesondere, wenn dafür eine Frequenz gewählt wird, die nicht der Arbeitsfrequenz entspricht, so kann bei laufender Applikation die Impedanz des Gewebes zwischen einzelnen Elektrodenpaaren gemessen werden, ohne dass der Applikationsstrom diese Messung wesentlich behindert. Dazu umfasst die Messeinrichtung einen Frequenzdiskriminator oder -filter, der in der Lage ist, die Frequenz der Impedanzmessung, beispielsweise 20 kHz, von der Arbeitsfrequenz, beispielsweise 470 kHz, zu trennen.

Die Figuren 5a und 5b zeigen ein weiteres Beispiel für die Anwendung der erfindungsgemäßen Applikationsvorrichtung. Statt in das Zielgewebe werden in diesem Beispiel drei Elektrodennadeln 100 knapp außerhalb des Zielgewebes punktiert, so dass das Zielgewebe sich zwischen den drei Elektrodennadein 100 befindet. Im Gegensatz zu dem in den Figuren 4a und 4b dargestellten Beispiel erfolgt der Stromfluss hier nicht parallel zu den Nadelachsen, sondern von einer Elektrodennadel 100 zur anderen. Dadurch lässt sich im Zielgewebe 200 ein homogener Stromfluss erzielen. Außerdem kann ein Verschleppen malignen Gewebes beim Entfernen der Elektrodennadeln vermieden werden.

Die Applikationsvorrichtung kann entweder ausschließlich mit axialem Stromfluss entlang der Nadeln, ausschließlich mit Stromfluss zwischen zwei verschiedenen Nadeln oder einer Kombination von beidem betrieben werden. Die Richtung des Stromflusses kann nach einer Neuauswahl der Untermenge aktiver Elektroden von dem einen Modus in den anderen Modus umgeschaltet werden.

Obwohl in den Ausführungsbeispielen jeweils Elektrodenmengen mit sechs Elektroden dargestellt sind, kann die Elektrodenmenge auch mehr oder weniger als sechs Elektroden enthalten.

## Patentansprüche

1. Applikationsvorrichtung zum Applizieren eines Hochfrequenzstroms zum thermischen Veröden von Körpergewebe, umfassend eine angeschlossene Elektrodenmenge (7) mit mindestens drei in Körpergewebe einführbaren aktiven Elektroden (1 - 6), einen Hochfrequenzgenerator (17) zum Erzeugen einer Hochfrequenzspannung, der umschaltbar mit einer oder mehreren der Elektroden (1 - 6) zu verbinden ist, und eine Messeinrichtung (13) zum Messen der Impedanz des Körpergewebes zwischen allen oder ausgewählten aktiven Elektroden (1 - 6) **gekennzeichnet durch** eine Auswahleinrichtung (15), die mit der Messeinrichtung (13) verbunden ist und dazu ausgestaltet ist, eine mindestens zwei aktive Elektroden (5, 6) umfassende Untermenge (19) aus der Elektrodenmenge (7) anhand der gemessenen Impedanz auszuwählen und **durch** eine Steuereinrichtung (11), die mit der Auswahleinrichtung (15) verbunden ist und zum Anlegen der Hochfrequenzspannung an die aktiven Elektroden (5, 6) der ausgewählten Untermenge (19) derart, dass zwischen ihnen ein Hochfrequenzstrom **durch** das Körpergewebe fließt, ausgestaltet ist.

2. Applikationsvorrichtung nach Anspruch 1, **dadurch gekennzeichnet, dass** die Messeinheit ausgebildet ist, aus einer gemessenen Impedanz den ohmschen Widerstand zwischen einer vorgegebenen Anzahl aktiver Elektroden (5, 6) einer Untermenge (19) aller angeschlossenen Elektroden abzuleiten.

3. Applikationsvorrichtung nach Anspruch 1 oder 2, **dadurch gekennzeichnet, dass** die Auswahleinrichtung (15) derart ausgestaltet ist, dass sie selbsttätig eine neue Impedanzmessung und eine neue Auswahl einer mindestens zwei Elektroden (5, 6) umfassenden Untermenge (19) durchführt, wenn seit der vorangegangenen Auswahl eine vorbestimmte Zeit verstrichen ist.

4. Applikationsvorrichtung nach einem der vorangehenden Ansprüche, **dadurch gekennzeichnet, dass** die Messeinrichtung (13) zum Messen der Impedanz oder des ohmschen Widerstandes des Körpergewebes zwischen den aktiven Elektroden (1 - 6) während der Applikation des Hochfrequenzstroms ausgelegt ist.

5. Applikationsvorrichtung nach Anspruch 4, **dadurch gekennzeichnet, dass** die Auswahleinrichtung (15) derart ausgestaltet ist, dass sie selbsttätig eine neue Auswahl einer mindestens zwei aktive Elektroden (5, 6) umfassenden Untermenge (19) durchführt, wenn die Impedanz, der ohmsche Widerstand, die Impedanzänderung oder die Änderung des ohmschen Widerstandes des Körpergewebes zwischen einer vorgegebenen Anzahl aktiver Elektroden (5, 6) der Untermenge einen vorbestimmten Wert erreicht oder überschreitet.

6. Applikationsvorrichtung nach einem der Ansprüche 1 bis 5, **dadurch gekennzeichnet, dass** die Auswahleinrichtung (15) derart ausgestaltet ist, dass sie vor der Auswahl bzw. der Neuauswahl der mindestens zwei aktive Elektroden (5, 6) umfassenden Untermenge (19) die Messeinrichtung (13) veranlasst, eine Messung der Impedanz oder des ohmschen Widerstandes des Körpergewebes zwischen allen möglichen Paaren aus aktiven Elektroden (1 - 6) durchzuführen, und als Untermenge (19) diejenigen aktiven Elektroden (1 - 6) auswählt, zwischen denen die Impedanz, der ohmsche Widerstand, die Impedanzänderung oder die Änderung des ohmschen Widerstandes am geringsten ist oder einen vorbestimmten Wert nicht überschreitet.

7. Applikationsvorrichtung nach einem der vorangehenden Ansprüche, **dadurch gekennzeichnet, dass** die Steuervorrichtung (11) derart ausgestaltet ist, dass zu Beginn der Applikation die Hochfrequenzspannung in einem vorgegebenen zyklischen Wechsel an die aktiven Elektroden (1 - 6) angelegt wird und die Auswahl der mindestens zwei aktive Elektroden (5, 6) umfassenden Untermenge (19) anhand der Impedanz oder des ohmschen Widerstandes zu einem spätem Zeitpunkt der Applikation einsetzt.

8. Applikationsvorrichtung nach einem der vorangehenden Ansprüche, **dadurch gekennzeichnet, dass** die Auswahleinrichtung (15) oder die Steuereinrichtung (11) derart ausgestaltet ist, dass sie Untermengen oder Elektrodenkombinationen, für die die Impedanz oder der ohmsche Widerstand einen vorgegebenen Maximalwert Rₘₐₓ überschreitet, abschaltet und im weiteren Verlauf nicht wieder berücksichtigt.

9. Applikationsvorrichtung nach Anspruch 8, **dadurch gekennzeichnet, dass** die Steuereinrichtung (11) ausgebildet ist, eine über eine jeweils ausgewählte Untermenge (19) vorgegebene abzugebende Maximalleistung Pₘₐₓ bei Überschreiten einer definierten Impedanz oder eines definierten ohmschen Widerstandes kleiner Rₘₐₓ auf ein vorgegebenes Maß zu reduzieren.

10. Applikationsvorrichtung nach einem der vorangehenden Ansprüche, **dadurch gekennzeichnet, dass** die Auswahleinrichtung (15) oder die Steuereinrichtung (11) derart ausgestaltet ist, dass sie Untermengen oder Elektrodenkombinationen, für die die Impedanz oder der ohmsche Widerstand einen vorgegebenen Minimalwert Rₘᵢₙ unterschreitet, abschaltet.

11. Applikationsvorrichtung nach einem der vorangehenden Ansprüche, **dadurch gekennzeichnet, dass** die Steuereinrichtung (11) eine über eine jeweils ausgewählte Untermenge (19) abgegebene Leistung nach dem Auswählen der Untermenge (19) zunächst geringer einstellt und innerhalb vorgegebener Zeit in einer oder mehreren Stufen oder gleitend auf eine vorgegebene Maximalleistung Pₘₐₓ erhöht.

12. Applikationsvorrichtung nach einem der vorangehenden Ansprüche, **dadurch gekennzeichnet, dass** die Auswahleinrichtung (15) derart ausgestaltet ist, dass sie als Untermenge (19) mindestens drei aktive Elektroden auswählt und die Steuereinrichtung (11) derart ausgestaltet ist, dass die aktiven Elektroden mit Hochfrequenzspannungen beaufschlagt werden, die jeweils um einen festen Phasenwinkel zueinander phasenverschoben sind.

13. Applikationsvorrichtung nach Anspruch 12, **dadurch gekennzeichnet, dass** die ausgewählte Untermenge (19) drei aktive Elektroden umfasst und die Phasenwinkel 120 Grad betragen.

14. Applikationsvorrichtung nach einem der vorangehende Ansprüche, **dadurch gekennzeichnet, dass** die aktiven Elektroden (1 - 6) an Elektrodennadeln (100) angeordnet sind.

15. Applikationsvorrichtung nach Anspruch 14, **dadurch gekennzeichnet**, das die Elektrodennadeln (100) bipolare Elektrodennadeln sind

16. Applikationsvorrichtung nach Anspruch 15, **dadurch gekennzeichnet, dass** die Elektrodennadeln (100) multipolare Elektrodennadeln sind.

17. Applikationsvorrichtung nach Anspruch 14, **dadurch gekennzeichnet, dass** die Elektrodennadeln (100) mit einer Isolierenden Umhüllung umgeben sind und aus der Umhüllung um vorbestimmte Längen ausfahrbar sind.

18. Applikationsvorrichtung nach einem der Ansprüche 14 bis 17, **dadurch gekennzeichnet, dass** die Elektrodennadein (100) Kanäle für ein Fluid zur Kühlung oder Heizung der Elektrodennadeln (100) aufweisen.

19. Applikationsvorrichtung nach Anspruch 18, **dadurch gekennzeichnet, dass** die Kanäle zu den aktiven Elektroden (1 - 6) führen und zum Zuführen von Kühlfluid geeignet sind.

20. Applikationsvorrichtung nach Anspruch 18 oder 19, **dadurch gekennzeichnet, dass** das Fluid eine entionisierte Flüssigkeit ist.

21. Applikationsvorrichtung nach Anspruch 1, **dadurch gekennzeichnet, dass** der Hochfrequenzgenerator, die Messeinrichtung, die Auswahleinrichtung und die Steuereinrichtung in einem oder mehreren Gehäusen zu einem Applikationsgenerator zusammengefasst sind, der Steckanschlüsse für die Elektroden und deren Zuleitungen aufweist.

## Claims

1. Application device for applying a high frequency current for the thermal sclerosis of body tissue, including a connected electrode set (7) with at least three active electrodes (1 - 6) which can be introduced into the body tissue, a high frequency generator (17) for producing a high frequency voltage, which generator is to be connected in a switchable manner to one or more of the electrodes (1 - 6), and a measuring device (13) for measuring the impedance of the body tissue between all or selected active electrodes (1-6), **characterised** a selection device (15) which is connected to the measuring device (13) and is configured to select a subset (19) including at least two active electrodes (5, 6) from the electrode set (7) on the basis of the measured impedance and by a control device (11), which is connected to the selection device (15) and is designed to apply the high frequency voltage to the active electrodes (5, 6) of the selected subset (19) in such a way that a high frequency current flows between them through the body tissue.

2. Application device according to claim 1, **characterised in that** the measuring unit is designed to derive from a measured impedance the ohmic resistance between a predefined number of active electrodes (5, 6) of a subset (19) of all connected electrodes.

3. Application device according to claim 1 or 2, **characterised in that** the selection device (15) is designed such that it automatically performs a new impedance measurement and a new selection of a subset (19) comprising at least two electrodes (5, 6), if a predetermined time period has elapsed since the preceding selection.

4. Application device according to one of the preceding claims, **characterised in that** the measuring device (13) is designed to measure the impedance or ohmic resistance of the body tissue between the active electrodes (1 - 6) during the application of the high frequency current.

5. Application device according to claim 4, **characterised in that** the selection device (15) is designed in such a way that it automatically performs a new selection of a subset (19) comprising at least two active electrodes (5, 6), if the impedance, the ohmic resistance, the change in impedance or the change in ohmic resistance of the body tissue between a predetermined number of active electrodes (5, 6) of the subset reaches or exceeds a predefined value.

6. Application device according to one of claims 1 to 5, **characterised in that** the selection device (15) is designed such that prior to the selection or new selection of the subset (19) comprising at least two active electrodes (5, 6) it causes the measuring device (13) to perform a measurement of the impedance or the ohmic resistance of the body tissue between all possible pairs of active electrodes (1 - 6), and selects as a subset (19) those active electrodes (1 - 6) between which the impedance, the ohmic resistance, the change in impedance or the change in ohmic resistance is at its smallest or does not exceed a predefined value.

7. Application device according to one of the preceding claims, **characterised in that** the control device (11) is designed such that at the beginning of the application the high frequency voltage is applied to the active electrodes (1 - 6) in a predetermined cyclical variation, and the selection of the subset (19) comprising at least two active electrodes (5, 6) is made on the basis of the impedance or ohmic resistance at a later time during the application.

8. Application device according to one of the preceding claims, **characterised in that** the selection device (15) or the control device (11) is designed such that it switches off subsets or electrode combinations, for which the impedance or ohmic resistance exceeds a predetermined maximum value Rₘₐₓ, and does not take it into account in the further procedure.

9. Application device according to claim 8, **characterised in that** the control device (11) is designed to reduce to a predetermined value a maximum power Pₘₐₓ which is to be delivered and which is predetermined by way of a respectively selected subset (19), when a defined impedance or a defined ohmic resistance smaller than Rₘₐₓ is exceeded.

10. Application device according to one of the preceding claims, **characterised in that** the selection device (15) or the control device (11) is designed in such a way that it switches off subsets or electrode combinations for which the impedance or ohmic resistance falls below a predefined minimum value Rₘᵢₙ.

11. Application device according to one of the preceding claims, **characterised in that** the control device (11) initially lowers power delivered by a respectively selected subset (19) after the selection of the subset (19), and increases it within a predetermined time period in one or more stages or continuously to a predetermined maximum power Pₘₐₓ.

12. Application device according to one of the preceding claims, **characterised in that** the selection device (15) is designed in such a way that as a subset (19) it selects at least three active electrodes and the control device (11) is designed in such a way that the active electrodes are acted upon with high frequency voltages which are respectively phase-shifted relative to each other by a fixed phase angle.

13. Application device according to claim 12, **characterised in that** the selected subset (19) comprises three active electrodes and the phase angles are 120 degrees.

14. Application device according to one of the preceding claims, **characterised in that** the active electrodes (1 - 6) are arranged on electrode needles (100).

15. Application device according to claim 14, **characterised in that** the electrode needles (100) are bipolar electrode needles.

16. Application device according to claim 15, **characterised in that** the electrode needles (100) are multipolar electrode needles.

17. Application device according to claim 14, **characterised in that** the electrode needles (100) are surrounded by an insulating cover and can be extended from the cover by predetermined lengths.

18. Application device according to one of claims 14 to 17, **characterised in that** the electrode needles (100) comprise channels for a fluid for cooling or heating the electrode needles (100).

19. Application device according to claim 18, **characterised in that** the channels lead to the active electrodes (1 - 6) and are suitable for supplying cooling fluid.

20. Application device according to claim 18 or 19, **characterised in that** the fluid is a deionised liquid.

21. Application device according to claim 1, **characterised in that** the high frequency generator, the measuring device, the selection device and the control device are combined in one or more housings to form an application generator, which comprises plug connections for the electrodes and the feed lines thereof.

## Revendications

1. Dispositif d'application destiné à appliquer un courant haute fréquence pour la sclérose thermique de tissus organiques, comprenant un ensemble connecté d'électrodes (7) comportant au moins trois électrodes actives (1 - 6) pouvant être introduites dans les tissus organiques, un générateur haute fréquence (17) destiné à produire une tension haute fréquence qui doit être relié à une ou plusieurs des électrodes (1 - 6) de manière commutable, et un dispositif de mesure (13) destiné à mesurer l'impédance du tissu organique entre toutes les électrodes actives (1 - 6) ou des électrodes actives sélectionnées (1 - 6), **caractérisé par** un dispositif de sélection (15), qui est relié au dispositif de mesure (13) et qui est conçu afin de sélectionner un sous-ensemble (19) de l'ensemble d'électrodes (7) par l'impédance mesurée, ce sous-ensemble contenant au moins deux électrodes actives (5, 6), et par un dispositif de commande (11), qui est relié au dispositif de sélection (15) et qui est conçu afin d'appliquer la tension haute fréquence aux électrodes actives (5, 6) du sous-ensemble (19) sélectionné de telle façon qu'un courant haute fréquence circule par le tissu organique entre les électrodes.

2. Dispositif d'application selon la revendication 1 **caractérisé en ce que** l'unité de mesure est conçue afin de dériver la résistance ohmique entre un nombre prédéfini d'électrodes actives (5,6) d'un sous-ensemble (19) de toutes les électrodes connectées à partir d'une impédance mesurée.

3. Dispositif d'application selon la revendication 1 ou 2 **caractérisé en ce que** le dispositif de sélection (15) est conçu de telle façon qu'il effectue de façon autonome une nouvelle mesure de l'impédance et une nouvelle sélection d'un sous-ensemble (19) comportant au moins deux électrodes (5,6), si un temps prédéterminé s'est écoulé depuis la sélection précédente.

4. Dispositif d'application selon l'une des revendications précédentes **caractérisé en ce que** le dispositif de mesure (13) est conçu afin de mesurer l'impédance ou de la résistance ohmique du tissu organique entre les électrodes actives (1 - 6) pendant l'application du courant haute fréquence.

5. Dispositif d'application selon la revendication 4 **caractérisé en ce que** le dispositif de sélection (15) est conçu de telle façon qu'il effectue de façon autonome une nouvelle sélection d'un sous-ensemble (19) comportant au moins deux électrodes actives (5, 6) si l'impédance, la résistance ohmique, la modification de l'impédance ou la modification de la résistance ohmique du tissu organique entre un nombre prédéfini d'électrodes actives (5, 6) du sous-ensemble atteint ou dépasse une valeur prédéterminée.

6. Dispositif d'application selon l'une des revendications 1 à 5 **caractérisé en ce que** le dispositif de sélection (15) est conçu de telle façon qu'il amène le dispositif de mesure (13) à effectuer une mesure de l'impédance ou de la résistance ohmique du tissu organique entre toutes les paires possibles d'électrodes actives (1 - 6) avant la sélection ou la nouvelle sélection du sous-ensemble (19) comportant au moins deux électrodes actives (5, 6), et qu'il sélectionne en tant que sous-ensemble (19) les électrodes actives (1 - 6) entre lesquelles l'impédance, la résistance ohmique, la modification de l'impédance ou la modification de la résistance ohmique est la plus faible ou ne dépasse pas une valeur prédéterminée.

7. Dispositif d'application selon l'une des revendications précédentes **caractérisé en ce que** le dispositif de commande (11) est conçu de telle façon qu'au début de l'application la tension haute fréquence est appliquée aux électrodes actives (1 - 6) selon une alternance cyclique prédéfinie et qu'il met en oeuvre la sélection du sous-ensemble (19) comportant au moins deux électrodes actives (5, 6) par l'impédance ou la résistance ohmique à un moment postérieur de l'application.

8. Dispositif d'application selon l'une des revendications précédentes **caractérisé en ce que** le dispositif de sélection (15) ou le dispositif de commande (11) est conçu de telle façon qu'il met hors tension des sous-ensembles ou des combinaisons d'électrodes pour lesquels l'impédance ou la résistance ohmique dépasse une valeur maximale Rₘₐₓ prédéfinie et qu'il ne les prend plus en compte par la suite.

9. Dispositif d'application selon la revendication 8 **caractérisée en ce que** le dispositif de commande (11) est conçu afin de réduire une puissance maximale Pₘₐₓ prédéfinie à attribuer à un sous-ensemble respectivement sélectionné (19) jusqu'à une valeur prédéterminée lorsqu'une impédance définie ou qu'une résistance ohmique définie dépasse la plus petite valeur de Rₘₐₓ.

10. Dispositif d'application selon l'une des revendications précédentes **caractérisé en ce que** le dispositif de sélection (15) ou le dispositif de commande (11) est conçu de telle façon qu'il met hors tension des sous-ensembles ou des combinaisons d'électrodes pour lesquels l'impédance ou la résistance ohmique est inférieure à une valeur minimale Rₘᵢₙ prédéfinie.

11. Dispositif d'application selon l'une des revendications précédentes **caractérisé en ce que** le dispositif de commande (11) règle d'abord une puissance à attribuer à un sous-ensemble respectivement sélectionné (19) sur une valeur faible et qu'il l'augmente en l'espace d'une durée prédéfinie en une ou plusieurs étapes ou par glissement jusqu'à atteindre une puissance maximale Pₘₐₓ prédéfinie.

12. Dispositif d'application selon l'une des revendications précédentes **caractérisé en ce que** le dispositif de sélection (15) est conçu de telle façon qu'il sélectionne au moins trois électrodes actives en tant que sous-ensemble (19) et **en ce que** le dispositif de commande (11) est conçu de telle façon que les électrodes actives sont soumises à des tensions haute fréquence qui sont respectivement déphasées les unes par aux autres selon un angle de phase fixe.

13. Dispositif d'application selon la revendication 12 **caractérisé en ce que** le sous-ensemble sélectionné (19) comporte trois électrodes actives et que les angles de phase sont de 120 degrés.

14. Dispositif d'application selon l'une des revendications précédentes **caractérisé en ce que** les électrodes actives (1 - 6) sont disposées sur des aiguilles à électrode (100).

15. Dispositif d'application selon la revendication 14 **caractérisé en ce que** les aiguilles à électrode (100) sont des aiguilles à électrode bipolaires.

16. Dispositif d'application selon la revendication 15 **caractérisé en ce que** les aiguilles à électrode (100) sont des aiguilles à électrode multipolaires.

17. Dispositif d'application selon la revendication 14 **caractérisé en ce que** les aiguilles à électrode (100) sont entourées d'une enveloppe isolante et qu'elles peuvent être sorties de l'enveloppe sur des longueurs prédéterminées.

18. Dispositif d'application selon l'une des revendications 14 à 17 **caractérisé en ce que** les aiguilles à électrode (100) comportent des canaux pour un fluide destiné à refroidir ou à chauffer les aiguilles à électrode (100).

19. Dispositif d'application selon la revendication 18 **caractérisé en ce que** les canaux conduisent aux électrodes actives (1 - 6) et sont adaptés pour la circulation d'un fluide de refroidissement.

20. Dispositif d'application selon la revendication 18 ou 19 **caractérisé en ce que** le fluide est un liquide désionisé.

21. Dispositif d'application selon la revendication 1 **caractérisé en ce que** le générateur haute fréquence, le dispositif de mesure, le dispositif de sélection et le dispositif de commande sont rassemblés dans un ou plusieurs boîtiers pour former un générateur d'application, qui comprend des connexions enfichables pour les électrodes et leurs conduites.
